# FASCICULE DE BREVET EUROPEEN

(11) **EP 0 753 562 B1**
(45) Date de publication et mention de la délivrance du brevet: **15.12.1999**
(21) Numéro de dépôt: 96401465.8
(22) Date de dépôt: 03.07.1996
(51) Int. Cl.: C10G 2/00, C07C 1/06

(54) **Procédé de conversion du gaz de synthèse en phase liquide**
Verfahren zur Umsetzung von Synthesegas in flüssiger Phase
Liquid phase conversion of synthesis gas

(30) Priorité: 13.07.1995 FR 8600037
(43) Date de publication de la demande: 15.01.1997
(73) Titulaire: INSTITUT FRANCAIS DU PETROLE, 92502 Rueil-Malmaison (FR)
(72) Inventeur: Chaumette, Patrick, 78380 Bougival (FR); Boucot, Pierre, 69360 Ternay (FR); Galtier, Pierre, 38209 Vienne Cedex (FR)

(56) Documents cités:
- EP-A- 0 188 304
- DE-C- 897 549
- GB-A- 728 543
- NL-A- 7 708 307
- US-A- 4 413 063
- US-A- 4 529 738

## Description

La présente invention concerne un procédé de synthèse d'hydrocarbures essentiellement C₅⁺ (c'est-à-dire d'hydrocarbures comportant au moins 5 atomes de carbone par molécule), utilisables en tant que carburant ou combustible liquide, à partir de gaz de synthèse.

Le gaz de synthèse est un mélange CO-(CO₂)-H₂, c'est-à-dire un mélange CO-H₂ de monoxyde de carbone (CO) et d'hydrogène (H₂) comprenant éventuellement du dioxyde de carbone (CO₂). La réaction de synthèse d'hydrocarbures à partir de gaz de synthèse, opérée généralement à une température comprise entre 150 et 350°C et sous pression, est connue sous le nom de synthèse Fischer-Tropsch. Les catalyseurs habituellement utilisés pour la transformation de mélanges CO-(CO₂)-H₂ en hydrocarbures liquides ou gazeux comprennent généralement au moins un métal du groupe VIII tel que le fer, le ruthénium, le cobalt ou le nickel.

Les produits préparés par synthèse Fischer-Tropsch en présence de ces catalyseurs métalliques présentent une distribution très large en terme de poids moléculaire. Ainsi seulement une faible proportion des produits obtenus se situe dans la gamme des distillats moyens constitués par des fractions kérosène et gasoil, la (ou les) fraction(s) kérosène étant constituée(s) par un mélange d'hydrocarbures dont les points d'ébullition sont approximativement compris entre 140 et 300°C, et la (ou les) fraction(s) gasoil étant constituée(s) par un mélange d'hydrocarbures dont les points d'ébullition sont approximativement compris entre 180 et 370°C lors d'une distillation atmosphérique telle que réalisée sur un brut pétrolier par l'homme du métier. La réaction que l'on cherche à réaliser dans le cadre de la présente demande est la réaction de synthèse d'hydrocarbures essentiellement C₅⁺ à partir de gaz de synthèse.

La synthèse Fischer-Tropsch est une réaction très exothermique. Si bien que lorsque le procédé pour la mise en oeuvre de ladite réaction de synthèse est opéré en phase gazeuse et avec un catalyseur en lit fixe, la conversion du monoxyde de carbone doit être limitée en deçà de 85%, afin d'éviter les instabilités thermiques dans le lit catalytique, ce qui oblige à séparer et recycler le gaz de synthèse non converti.

Un autre procédé qui a été envisagé pour la mise en oeuvre de la réaction consiste à opérer en présence d'une phase liquide et avec un catalyseur en suspension (réacteur à lit circulant également appelé "slurry"). Dans ce cas, la conversion du CO peut atteindre, voire dépasser 95%. Par contre, le catalyseur mis en suspension et en circulation avec le liquide inerte, doit être séparé des produits de la réaction, puis recyclé.

Un autre procédé a été également envisagé qui consiste à opérer en présence d'une phase liquide circulant de haut en bas, en mélange avec le gaz de synthèse, et d'un catalyseur en lit fixe. Ainsi le brevet US-A-4.413.063 revendique un procédé permettant de synthétiser des hydrocarbures ou des alcools à partir de gaz de synthèse en présence d'un catalyseur et d'un diluant inerte, le gaz de synthèse circulant vers le bas en mélange avec la phase liquide et à travers le catalyseur en lit fixe. Le réacteur à lit fixe avec écoulements descendants du liquide et du gaz, également appelé "trickle bed downflow", est une solution qui permet d'éviter la circulation et la séparation du catalyseur tout en limitant les instabilités thermiques.

L'objet de la présente invention est de proposer un procédé qui évite les désavantages des procédés en lit fixe/phase gazeuse et en lit circulant, et qui permet de conserver les avantages d'un réacteur à lit fixe avec écoulements descendants du liquide et du gaz, tout en favorisant plus particulièrement la formation d'hydrocarbures C₅⁺ par rapport à la formation de méthane.

Le procédé de la présente invention est un procédé de synthèse d'hydrocarbures essentiellement linéaires et saturés C₅⁺ à partir de gaz de synthèse, la phase gazeuse réactive circulant soit dans le sens descendant, soit dans le sens ascendant à travers une zone de réaction comprenant un lit fixe de catalyseur, ledit procédé étant caractérisé en ce qu'il comprend la circulation ascendante à travers ladite zone d'une phase liquide inerte, avec une vitesse superficielle supérieure à 0,01 cm/s, de préférence supérieure à 0,1 cm/s et de manière encore plus préférée supérieure à 1 cm/s. Le procédé selon l'invention est également appelé "trickle bed upflow".

La vitesse superficielle de la phase liquide inerte est définie comme le rapport entre la vitesse volumique horaire, dans les conditions de température et de pression sélectionnées pour la réaction, et la superficie de la coupe transversale de la zone de réaction, ladite zone étant considérée sans catalyseur. La vitesse superficielle optimale dépend en partie de la taille des particules de catalyseur et des propriétés physico-chimiques du liquide. Elle semble pratiquement indépendante de la vitesse superficielle du gaz.

La zone de réaction comporte un ou plusieurs réacteurs, chaque réacteur comprenant au moins un lit fixe de catalyseur. Dans le cas où la zone de réaction comprend plusieurs réacteurs, ceux-ci peuvent être placés en série ou en parallèle. Dans tous les cas, la configuration de la zone de réaction s'effectue de façon bien connue par l'homme du métier.

Les particules de catalyseur ont généralement un diamètre moyen compris entre 0,2 et 10 mm, de manière préférée entre 0,5 à 6 mm et de manière plus préférée entre 1 et 3 mm.

Le catalyseur peut être tout type de catalyseur efficace dans la réaction de synthèse d'hydrocarbures à partir de gaz de synthèse. Par exemple tout catalyseur à base de fer ou de cobalt supporté ou non, tels que ceux décrits dans la demande de brevet européen EP-A-0.581.619 et dans le brevet français FR-A-2.677.992.

La phase liquide ne prend pas part à la réaction et n'a aucun effet néfaste sur cette dernière. Il s'agit préférentiellement d'une coupe hydrocarbonée, de manière encore plus préférée comprenant essentiellement entre 10 et 20 atomes de carbone par molécule, telle que une coupe gasoil ou une coupe kérosène. Si le catalyseur est sensible au soufre, une coupe hydrocarbonée désulfurée est préférentiellement utilisée. La phase liquide est de préférence partiellement vaporisable dans les conditions de la réaction, de façon à éliminer les calories dégagées par la réaction.

Dans un mode de réalisation préféré de l'invention, la phase liquide comprend au moins un produit partiellement vaporisable, par exemple entre 0 et 80% d'un produit au moins partiellement vaporisable. Ainsi les évacuations de calories et les transferts de chaleur au sein de la zone de réaction sont améliorés. On entend par 'vaporisable' tout produit liquide qui, dans les conditions de la réaction, est pratiquement complètement sous forme de gaz. On entend par 'produit partiellement vaporisable' un produit dont une partie, généralement entre 10 et 100 %, est vaporisable. De façon préférée selon le présent mode de réalisation de l'invention, la phase liquide comprend une coupe hydrocarbonée partiellement vaporisable, par exemple une coupe hydrocarbonée comprenant des hydrocarbures contenant 5, 6, 7, 8, 9 ou 10 atomes de carbone par molécule.

Dans un mode de réalisation préféré selon l'invention, indépendemment ou non du mode de réalisation précédent, la phase liquide inerte est avantageusement obtenue en recyclant une partie d'une fraction des hydrocarbures produits par la réaction ; de préférence ladite fraction est la fraction gasoil ou kérosène des hydrocarbures produits par la réaction. Dans ce cas, la phase liquide inerte qui est introduite initialement dans la zone de réaction est apportée de l'extérieur (par opposition à une phase liquide produite dans la zone de réaction Fischer-Tropsch, c'est-à-dire à l'intérieur), puis ladite phase liquide comprend une partie d'une fraction des hydrocarbures produits par la réaction qui est recyclée dans ladite zone. De préférence ladite fraction est la fraction gasoil ou la fraction kérosène.

A titre indicatif, la phase liquide inerte a généralement une densité comprise entre 0,2 et 2,5 g/cm³ et une viscosité comprise entre 0,05 et 10 centipoises (0,05 à 10 mPa.s) dans les conditions de la réaction, mais ces valeurs ne sont en aucun cas obligatoires.

Les meilleurs résultats sont obtenus moyennant une bonne distribution du liquide en bas de la zone de réaction, au moyen d'équipements connus de l'homme de métier, par exemple un plateau distributeur horizontal présentant de nombreuses perforations.

Le procédé selon l'invention est particulièrement bien adapté pour être utilisé en tant que procédé de fabrication à partir d'un gaz de synthèse d'un mélange d'hydrocarbures esssentiellement linéaires et saturés, contenant généralement au moins 80% en poids, par rapport à l'ensemble des hydrocarbures formés, d'une coupe comprenant des hydrocarbures C₅⁺ et de préférence moins de 10% poids d'oléfines dans ladite coupe C₅⁺. Le procédé selon l'invention permet donc d'obtenir des hydrocarbures essentiellement paraffiniques, dont la fraction présentant les points d'ébullition les plus élevés peut être convertie avec un rendement élevé en distillats moyens (coupes gasoil et kérosène) par un procédé d'hydroconversion tel que l'hydrocraquage et/ou l'hydroisomérisation catalytique(s).

Les conditions opératoires dans lesquelles sont menées la réaction dépendent de la nature du catalyseur et sont généralement les suivantes.

La conversion du gaz de synthèse en hydrocarbures est généralement opérée sous une pression totale comprise entre 0,1 et 15 MPa, de préférence entre 0,5 et 10 MPa, la température étant comprise entre 150 et 350 °C, de préférence entre 180 et 270°C.

La vitesse volumique horaire est habituellement comprise entre 100 et 20 000 volumes de gaz de synthèse par volume de catalyseur et par heure et de préférence entre 400 et 10 000 volumes de gaz de synthèse par volume de catalyseur et par heure.

Le rapport molaire H₂/CO dans le gaz de synthèse est généralement compris entre 0,5 et 5, de préférence entre 1,2 et 3,5.

Dans la mise en oeuvre du procédé selon l'invention, le catalyseur est tout d'abord chargé dans la zone de réaction et préréduit par mise en contact avec au moins un composé réducteur, par exemple l'hydrogène pur ou un mélange de gaz réducteurs tels que l'hydrogène et/ou le monoxyde de carbone, et éventuellement au moins un gaz inerte tel que l'azote, le rapport molaire (composé réducteur):(composé réducteur + gaz inerte) étant compris entre 0,001:1 et 100:1 dans le cas où au moins un gaz inerte est présent. La préréduction est généralement menée entre 150 et 600° C, de préférence entre 200 et 500° C, entre 0,1 et 10 MPa, et à une vitesse volumétrique horaire de 100 à 40 000 volumes de mélange par volume de catalyseur et par heure. Cette préréduction est éventuellement menée en phase liquide, la phase liquide de la préréduction étant par exemple constituée d'au moins un hydrocarbure comprenant au moins 5 atomes de carbone par molécule.

Les exemples qui suivent illustrent l'invention sans toutefois en limiter la portée.

### Exemples:

Le catalyseur utilisé dans les exemples 1 à 4 est préparé de la façon suivante:

A une solution contenant 40 g de nitrate de cobalt, 1g de trichlorure de ruthénium héxamine et 0,3 g de nitrate de cuivre trihydraté dissous dans 50 ml d'eau, sont ajoutés progressivement et simultanément 75 g de solution colloïdale de silice à 40 % poids de SiO₂ (Ludox 40) et 2ml d'acide nitrique à 10%, de façon à maintenir le pH entre 1 et 2. La solution est maintenue sans agitation pendant 10mn; puis 25g de LUDOX AS40 sont ajoutés, le pH évoluant pour se stabiliser entre 5,5 et 6,5 unités pH. Après 12mn, un gel de silice contenant les sels de cobalt, cuivre, et ruthénium est formé.

Le gel obtenu est séparé des eaux mères par filtration, lavé à l'eau, séché à l'étuve entre 40 et 120°C, puis calciné à 450°C et mis en forme par pastillage. les pastilles de taille 5x5mm sont ensuite recalcinées à 600°C.

Le catalyseur est réduit dans le réacteur, préalablement à la synthèse d'hydrocarbures, au moyen d'un mélange contenant 6% d'hydrogène dans l'azote, jusqu'à 240°C, puis par de l'hydrogène pur jusqu'à 500°C, à la pression atmosphérique.

### Exemple 1 (selon l'invention):

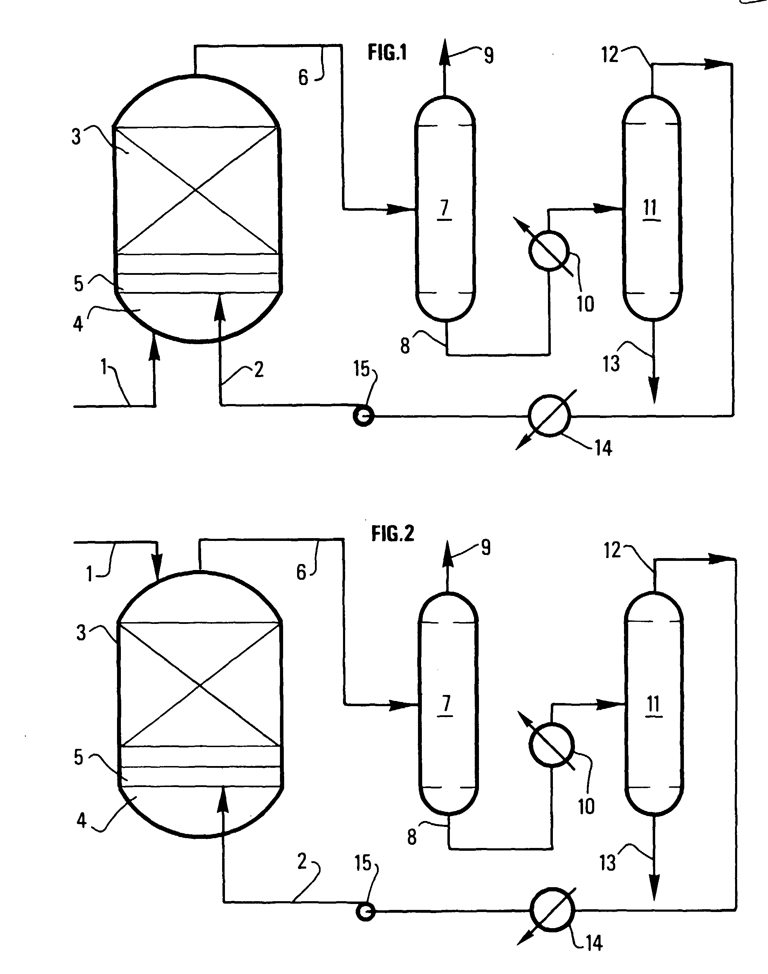

Dans cet exemple illustré par la figure 1, le gaz de synthèse (mélange H₂ + CO, conduite 1) et la phase liquide (conduite **2**) circulent de bas en haut et passent à travers 1,2 dm3 du catalyseur (**3**) mentionné ci-dessus et disposé en lit fixe dans le réacteur **4**. Le liquide est distribué uniformément à travers une plaque perforée (**5**). L'effluent est évacué au moyen de la conduite (**6**). Les paraffines liquides sont séparées de la phase gaz, à la sortie du réacteur, dans le séparateur (**7**), et envoyées en continu par la ligne (**8**) vers le réchauffeur (**10**), jusqu'au séparateur **11**. Le gaz issu du séparateur **7** est évacué au moyen de la conduite **9** et analysé par chromatographie. Dans le séparateur **11**, les paraffines lourdes synthétisées dans le réacteur **4** sont séparées puis récupérées par la conduite **13** ; elles sont analysées par chromatographie. Par la conduite **12** une phase gazeuse est récupérée qui, après passage dans le condenseur **14**, se condense en la phase liquide recyclée au réacteur **4** via la pompe **15** et la conduite **2**.

Le réacteur a un diamètre de 4 cm et une hauteur de 1 mètre. Le gaz de synthèse utilisé pour la synthèse d'hydrocarbures consiste en un mélange contenant 66,7% d'hydrogène et 33,3% de monoxyde de carbone. Le gaz est introduit avec un débit de 1,2 m³/h, soit une V.V.H. gaz (vitesse volumique horaire) de 1000 h⁻¹. La réaction est opérée à 220°C et 2MPa.

La phase liquide est une coupe paraffinique C₁₀-C₁₆ ne contenant pas de soufre qui est introduite au démarrage de l'unité, puis séparée des effluents à la sortie du réacteur, et ensuite recyclée. Le débit de cette phase liquide est d'environ 200 l/h à la température de réaction, soit une vitesse spatiale de 4,5 cm/s. Les produits plus légers ou plus lourds que cette phase liquide, ainsi que l'eau coproduite dans la réaction, sont séparés, évacués et analysés.

Si l'on nomme C1, C2, C3, ... Cn le nombre de molécules-grammes de monoxyde de carbone CO et de dioxyde de carbone CO₂ (si CO₂ est présent) transformées en hydrocarbures contenant de 1 à n atomes de carbone par molécule; le nombre Nc d'atomes-grammes de carbone dans les produits formés lors de la réaction peut être calculé selon la formule suivante : Nc = C1 + 2 C2 + 3 C3 + ..... + nCn.

Alors :
- la conversion est définie comme le rapport entre le nombre Nc et le nombre de moles de CO et de CO₂ (si CO₂ est présent) dans la charge, ledit rapport étant exprimé en pourcentage
- la sélectivité en méthane CH₄ est définie comme le rapport entre le nombre C1 et le nombre Nc, ledit rapport étant exprimé en pourcentage
- la sélectivité en C₅⁺ est définie comme le rapport entre (5 C5 + 6 C6 + ... + n Cn) et Nc, ledit rapport étant exprimé en pourcentage.

Dans ces conditions, dans le présent exemple 1, la conversion est de 72 %, la sélectivité en méthane est de 8%, et la sélectivité en hydrocarbures C₅⁺ est de 86%. (voir tableau 1).

### Exemple 2 (selon l'invention):

La réaction est effectuée dans des conditions identiques à celles de l'exemple 1 excepté pour la vitesse spatiale de la phase liquide qui est de 1 cm/s.

Dans ces conditions, la conversion est de 80%, la sélectivité en méthane est de 5%, et la sélectivité en hydrocarbures C₅⁺ est de 90%. (voir tableau 1)

### Exemple 3 (selon l'invention):

La réaction est effectuée dans des conditions identiques à celles de l'exemple 1 excepté pour la phase gaz qui circule de haut en bas avec la même V.V.H. égale à 1000 h-1. Ainsi, dans cet exemple illustré par la figure 2, le gaz de synthèse (mélange H₂ + CO, conduite 1) circule de haut en bas et la phase liquide (conduite **2**) circule de bas en haut; les deux phases passent à travers 1,2 dm3 du catalyseur (**3**) mentionné ci-dessus et disposé en lit fixe dans le réacteur **4**. Les autre références de la figure 2 sont celles de la figure 1.

Dans ces conditions, la conversion est de 72%, la sélectivité en méthane est de 6%, et la sélectivité en hydrocarbures C₅⁺ est de 88%. (voir tableau 1)

### Exemple 4 (comparatif):

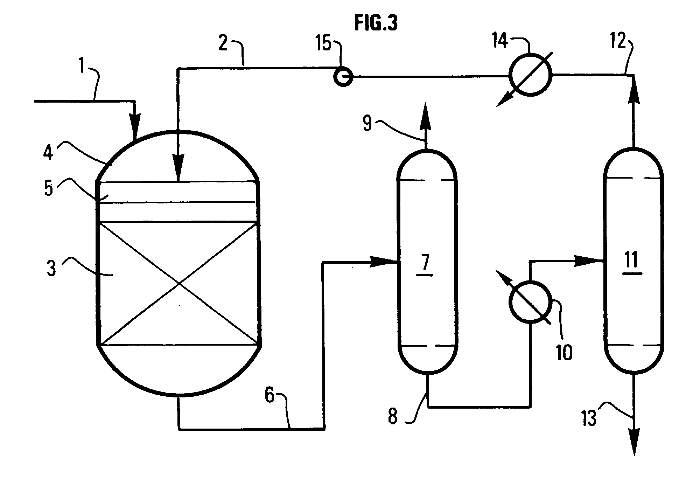

La réaction est effectuée dans des conditions identiques à celles de l'exemple 1 excepté pour les phases liquides et gaz qui circulent toutes les deux de haut en bas. Ainsi, dans cet exemple illustré par la figure 3, le gaz de synthèse (mélange H₂ + CO, conduite **1**) et la phase liquide (conduite **2**) circulent toutes les deux de haut en bas et passent à travers 1,2 dm3 du catalyseur (**3**) mentionné ci-dessus et disposé en lit fixe dans le réacteur **4**. Les autre références de la figure 3 sont celles de la figure 1.

Dans ces conditions, la conversion est de 77%, la sélectivité en méthane est de 21%, et la sélectivité en hydrocarbures C₅⁺ est de 68%.(voir tableau 1)

**Tableau 1**

| | Conversion | Sélectivité en CH₄ | Sélectivité en C₅⁺ |
|---|---|---|---|
| Ex. 1 (liquide ascendant, gaz ascendant) | 72 % | 8 % | 86 % |
| Ex. 2 (liquide ascendant, gaz ascendant) | 80 % | 5 % | 90 % |
| Ex. 3 (liquide ascendant, gaz descendant) | 72 % | 6 % | 88 % |
| Ex. 4 comparatif (liquide descendant, gaz descendant) | 77 % | 21 % | 68 % |

## Revendications

1. Procédé de synthèse d'hydrocarbures essentiellement linéaires et saturés C₅⁺ à partir de gaz de synthèse, la phase gazeuse réactive circulant soit dans le sens descendant, soit dans le sens ascendant à travers une zone de réaction comprenant au moins un lit fixe de catalyseur, ledit procédé étant caractérisé en ce qu'il comprend la circulation ascendante à travers ladite zone d'une phase liquide inerte, avec une vitesse superficielle supérieure à 0,01 cm/s.

2. Procédé selon la revendication 1 tel que les particules de catalyseur ont un diamètre moyen compris entre 0,2 et 10 mm.

3. Procédé selon l'une des revendications 1 ou 2 tel que la phase liquide est une coupe hydrocarbonée.

4. Procédé selon la revendication 3 tel que ladite coupe comprend essentiellement des hydrocarbures comportant entre 10 et 20 atomes de carbone par molécule.

5. Procédé selon l'une des revendications 1 à 4 tel que la phase liquide est une coupe gasoil ou une coupe kérosène.

6. Procédé selon l'une des revendications 1 à 5 tel que la phase liquide comprend au moins un produit partiellement vaporisable.

7. Procédé selon la revendication 6 tel que la phase liquide comprend une coupe hydrocarbonée comprenant des hydrocarbures contenant 5, 6, 7, 8, 9 ou 10 atomes de carbone par molécule.

8. Procédé selon l'une des revendications 1 à 7 tel que la conversion du gaz de synthèse en hydrocarbures est opérée sous une pression totale comprise entre 0,1 et 15 MPa, la température étant comprise entre 150 et 350 °C, la vitesse volumique horaire étant comprise entre 100 et 20 000 volumes de gaz de synthèse par volume de catalyseur et par heure, et le rapport molaire H₂/CO dans le gaz de synthèse étant compris entre 0,5 et 5.

9. Procédé selon l'une des revendications 1 à 8 tel que le catalyseur est préalablement préréduit au sein de la zone de réaction.

10. Procédé selon l'une des revendications 1 à 9 tel que la phase liquide inerte est obtenue en recyclant une partie d'une fraction des hydrocarbures produits par la réaction.

## Patentansprüche

1. Verfahren zur Synthese von im wesentlichen linearen und gesättigten C₅+ Kohlenwasserstoffen, ausgehend von Synthesegas, wobei die reaktive gasförmige Phase entweder in absteigender Richtung oder in aufsteigender Richtung durch eine Reaktionszone zirkuliert, die wenigstens ein festes Katalysatorbett umfaßt, wobei das Verfahren sich dadurch auszeichnet, daß es die aufsteigende Zirkulation durch diese Zone einer flüssigen inerten Phase mit einer Oberflächengeschwindigkeit oberhalb 0,01 cm/s umfaßt.

2. Verfahren nach Anspruch 1, derart, daß die Katalysatorpartikel einen mittleren Durchmesser zwischen 0,2 und 10 mm haben.

3. Verfahren nach einem der Ansprüche 1 oder 2, derart, daß die flüssige Phase eine kohlenwasserstoffhaltige Fraktion ist.

4. Verfahren nach Anspruch 3, derart, daß die Fraktion im wesentlichen Kohlenwasserstoffe mit 10 bis 20 Kohlenstoffatomen pro Molekül umfaßt.

5. Verfahren nach einem der Ansprüche 1 bis 4, derart, daß die flüssige Phase eine Gasölfraktion oder eine Kerosinfraktion ist.

6. Verfahren nach einem der Ansprüche 1 bis 5, derart, daß die flüssige Phase wenigstens ein teilweise verdampfbares Produkt umfaßt.

7. Verfahren nach Anspruch 6, derart, daß die flüssige Phase eine kohlenwasserstoffhaltige Fraktion umfaßt, welche Kohlenwasserstoffe umfaßt, die 5, 6, 7, 8, 9 oder 10 Kohlenstoffatome pro Molekül enthalten.

8. Verfahren nach einem der Ansprüche 1 bis 7, derart, daß die Umwandlung des Synthesegases in Kohlenwasserstoffe unter einem Gesamtdruck zwischen 0,1 und 15 MPa durchgeführt wird, die Temperatur zwischen 150 und 350°C liegt, die stündliche Volumengeschwindigkeit zwischen 100 und 20000 Volumenteilen Synthesegas pro Volumenteile Katalysator und Stunde beträgt und das Molverhältnis H₂/CO im Synthesegas zwischen 0,5 und 5 liegt.

9. Verfahren nach einem der Ansprüche 1 bis 8, derart, daß der Katalysator vorher mitten in der Reaktionszone vorreduziert wird.

10. Verfahren nach einem der Ansprüche 1 bis 9, derart, daß die flüssige inerte Phase erhalten wird, indem man einen Teil einer Fraktion der durch die Reaktion erzeugten Kohlenwasserstoffe recycliert.

## Claims

1. A process for the synthesis of essentially linear saturated C₅⁺ hydrocarbons from synthesis gas, the reactive gas phase circulating either as a downflow or as an upflow through a reaction zone which comprises a fixed catalyst bed, the process being characterized in that it comprises an upflow of an inert liquid phase through said zone at a superficial flow rate of more than 0.01 cm/s.

2. A process according to claim 1, in which the catalyst particles have a diameter in the range 0.2 mm to 10 mm.

3. A process according to claim 1 or claim 2, in which the liquid phase is a hydrocarbon cut.

4. A process according to claim 3, in which said cut essentially comprises hydrocarbons containing 10 to 20 carbon atoms per molecule.

5. A process according to any one of claims 1 to 4, in which the liquid phase is a gas oil cut or a kerosine cut.

6. A process according to any one of claims 1 to 5, in which the liquid phase comprises at least one partially vaporisable product.

7. A process according to claim 6, in which the liquid phase comprises a hydrocarbon cut comprising hydrocarbons containing 5, 6, 7, 8, 9 or 10 carbon atoms per molecule.

8. A process according to any one of claims 1 to 7, in which conversion of the synthesis gas to hydrocarbons is carried out at a total pressure in the range 0.1 MPa to 15 MPa, a temperature in the range 150°C to 350°C, an hourly space velocity in the range 100 to 20000 volumes of synthesis gas per volume of catalyst per hour, and a H₂/CO molar ratio in the synthesis gas in the range 0.5 to 5.

9. A process according to any one of claims 1 to 8, in which the catalyst is pre-reduced in the reaction zone.

10. A process according to any one of claims I to 9, in which the inert liquid phase is obtained by recycling a portion of a fraction of the hydrocarbons produced by the reaction.
